# EUROPEAN PATENT SPECIFICATION

(11) **EP 2 822 637 B1**
(45) Date of publication and mention of the grant of the patent: **01.08.2018**
(21) Application number: 13720624.9
(22) Date of filing: 08.03.2013
(51) Int. Cl.: A61M 25/10

(54) **MEDICAL BALLOON WITH MULTI-POSITION ACTUATOR FOR PRECISELY ARRANGING THE WORKING SURFACE**
MEDIZINISCHER BALLON MIT MEHRSTUFIGEM AKTUATOR ZUR GENAUEN ANORDNUNG DER ARBEITSFLÄCHE
BALLONNET MÉDICAL POURVU D'UN ACTIONNEUR À POSITIONS MULTIPLES POUR CONFIGURER AVEC PRÉCISION LA SURFACE DE TRAVAIL

(30) Priority: 09.03.2012 US 201261608917 P; 09.03.2012 NL 2008452; 31.12.2012 US 201261747444 P
(43) Date of publication of application: 14.01.2015
(73) Proprietor: Clearstream Technologies Limited, Enniscorthy, County Wexford (IE)
(72) Inventor: JENSEN, Angela, Kay, Tempe, AZ 85281 (US); SCHAFFER, Andrew, Tempe, AZ 85281 (US); STAPLETON, Corey, E., Gilbert, AZ 85296-8607 (US)
(74) Representative: Hoffmann Eitle
(86) International application number: PCT/US2013/029987
(87) International publication number: WO 2013/134701

(56) References cited:
- EP-A1- 0 850 659
- WO-A2-2007/054364
- US-A1- 2004 243 156
- US-A1- 2006 287 665
- US-A1- 2008 249 464
- US-A1- 2011 029 048
- US-A1- 2011 230 700

## Description

The following U.S. Patent Applications are disclosing a similar device; 61/608,852; 61/608,859; 61/608,862; 61/608,897; 61/608,902; 61/608,908; 61/608,913; 61/608,917; 61/608,927; 61/608,932; 61/608,941; and 61/747,444.

### Technical Field

This disclosure relates generally to balloons for performing medical procedures, such as angioplasty and, more particularly, to a medical balloon having a predetermined portion, such as a working surface or folds, that may be precisely actuated during use. Such a device is disclosed in the US20060287665.

### Background of the Invention

Balloons are routinely used to resolve or address flow restrictions or perhaps even complete blockages in tubular areas of the body, such as arteries or veins. In many clinical situations, the restrictions are caused by hard solids, such as calcified plaque, and require the use of high pressures to compact such blockages. Commercially available balloons employ complex technology to achieve high pressure requirements without sacrificing the profile of the balloon. Besides high pressure requirements, the balloons should also be resistant to puncture, easy to track and push, and present a low profile, especially when used for angioplasty.

In clinical practice, angioplasty balloons are expanded from a deflated, folded state to an expanded state within a vessel to treat a target area, such as a portion of the circumferential inner wall I of a blood vessel V, as shown in Figures 1 and 2. The inflation is traditionally completed using an X-ray contrast agent to provide better visibility under X-ray or other form of radiography during the interventional procedure, as illustrated in Figures 3 and 3a (which shows a balloon 12 with a balloon wall 28 bounding a contrast media CM having a width DX and bombarded with radiation R over a fluroscopic detector plate FDP and a graph illustrating the resulting image that may be obtained). Typically, a 70/30 percent mixture of contrast agent and saline is used to inflate the balloon during an angioplasty procedure.

The physician performing the angioplasty procedure should be able to locate the position of the uninflated balloon with accuracy, so that the balloon will be properly positioned once inflated. This is conventionally accomplished by attaching marker bands on the catheter shaft in the region corresponding to the balloon working surface. This "working surface" is the surface along the portion of the balloon that is used to achieve the desired treatment effect, such as contacting the calcified plaque (which surface in the case of a balloon having conical or tapering sections at the proximal and distal ends is typically co-extensive with a generally cylindrical barrel section).

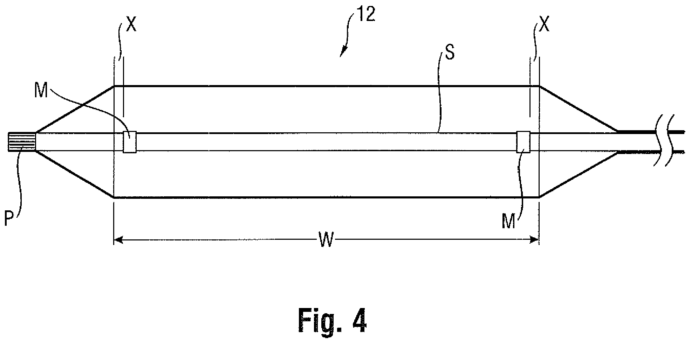

Misalignment of the marker bands during placement along the shaft sometimes results in their failure to correspond precisely to the extent of the working surface, as is shown in Figure 4 (note misalignment amount X between each interior marker band M carried by shaft S and working surface W of balloon 12, which also typically includes a radiopaque tip P at the distal end). Even upon exercising great care to position the markers properly on the underlying shaft in alignment with anticipated boundaries of the working surface when the balloon is inflated, there remains a tendency for mismatch due to several possible factors. One such factor may be the tolerance stack-ups arising as a consequence of the affixation of the balloon to the distal end of the catheter shaft. The balloon also has a tendency to grow in the longitudinal direction when inflated, especially with large and particularly long balloons. Another factor is the tendency of the portion of the catheter shaft within the balloon to bend or flex during inflation. This may lead to misalignment between radiopaque markers fixed to the shaft and the working surface.

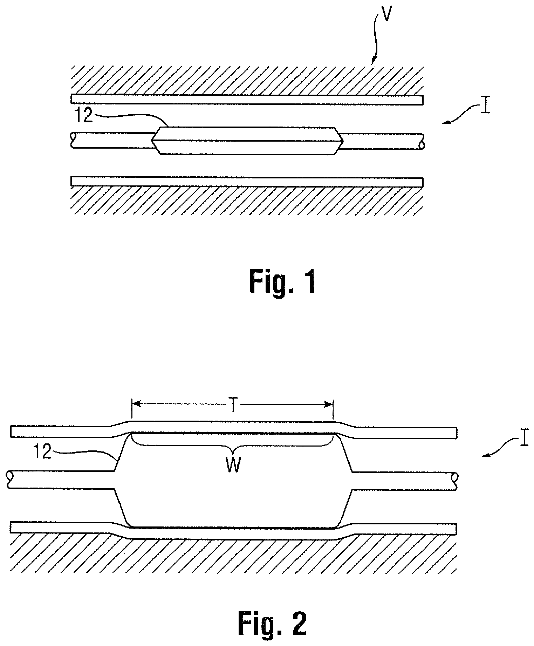

Whatever the cause, the resulting misalignment may prevent the clinician from accurately identifying the location of the working surface of the balloon during an interventional procedure. This may lead to a geographic misplacement, or "miss," of the intended contact between the target area T and the working surface W of the balloon 12 (see Figure 2). It is especially desirable to avoid such an outcome when the balloon is designed to deliver a payload (such as a therapeutic agent (e.g., a drug, such as paclitaxel, rapamycin, heparin and the like), a stent, a stent graft, or combinations of the foregoing) or a working element to a specified location within the vasculature, since a miss may prolong the procedure (such as, for example, by requiring redeployment of the balloon 12 or the use of another balloon catheter in the case of a drug coated balloon).

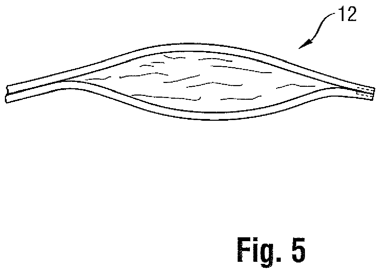

Upon deflation, the balloon may also be subject to a phenomenon known as "pancaking." In this condition, the balloon 12 folds down upon itself to a flattened state, as shown in Figure 5. This situation may cause the balloon to be viewed through fluoroscopy as perhaps still being in the inflated condition, since the full width of the balloon may still be perceived. This can give the clinician the false perception that the balloon remains inflated, when in fact it is not.

Accordingly, the need is identified for a balloon for which the working surface may be identified during an interventional procedure with enhanced precision. The solution would take into account the possible mismatch between fixed locations on the catheter shaft and the balloon to define the working surface, and would operate independent of the position of the portion of the catheter shaft within the balloon. The balloon may also be designed to fold in a preferential manner, which may help to avoid it from assuming undesirable configurations. Overall, procedural efficiency would be enhanced without remarkably increasing cost or complexity, and in a manner that can be applied to many existing catheter technologies without extensive modification.

### Summary of the Invention

An object of the disclosure is to provide a balloon as further disclosed in claim 1, for which the working surface may be identified during an interventional procedure with enhanced precision.

A further object of the disclosure is to provide a balloon that folds in a preferential manner.

### Brief Description of the Drawings

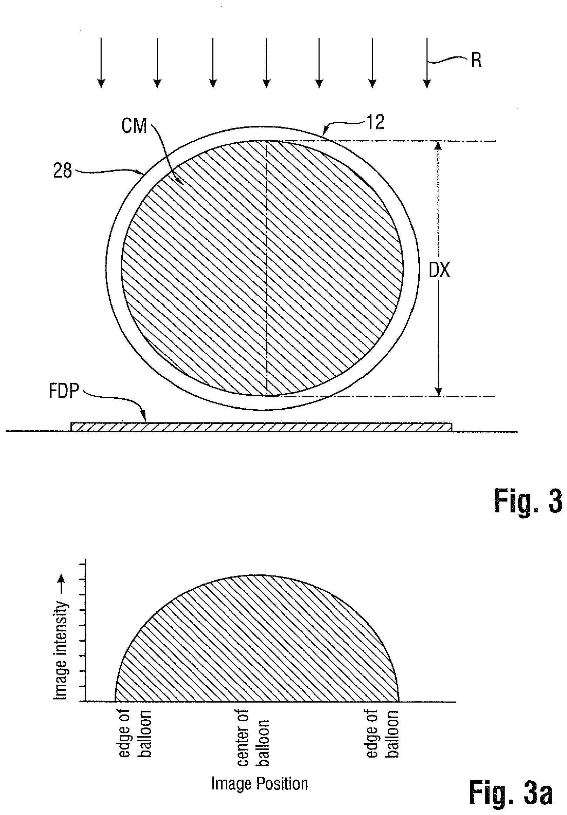

Figures 1-9 are illustrative of the background of the invention;

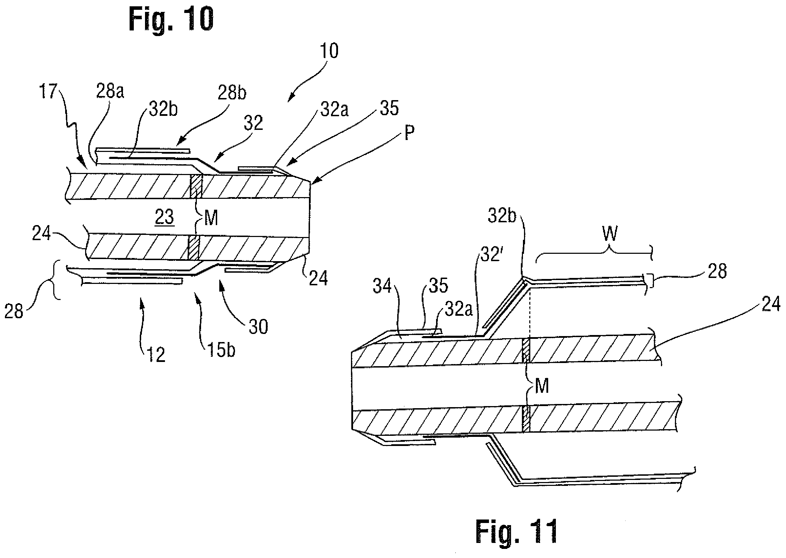

Figures 10 and 11 illustrate a first embodiment according to the disclosure;

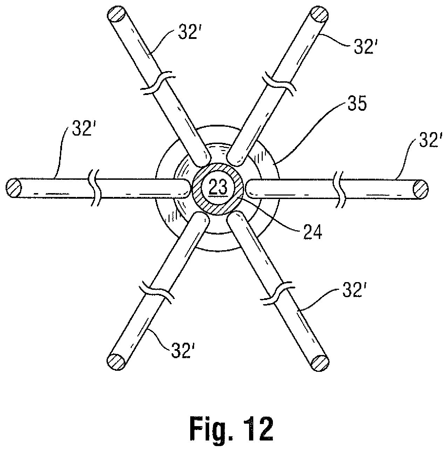

Figure 12 illustrates another aspect of the disclosure;

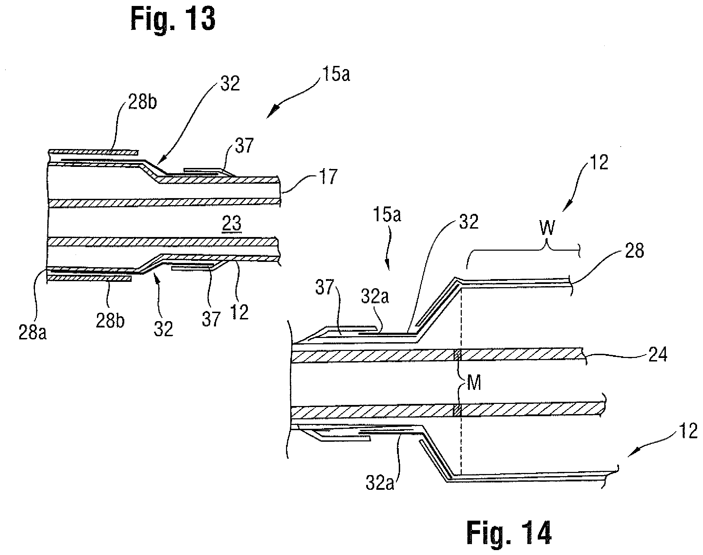

Figures 13 and 14 illustrate a second embodiment according to the disclosure;

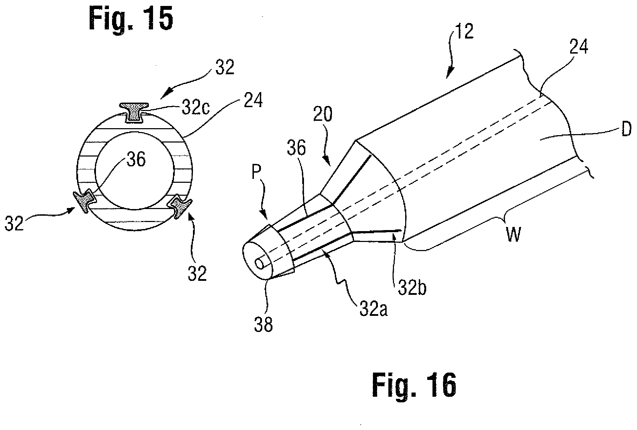

Figures 15, 16 and 17 illustrate a third embodiment according to the disclosure; and

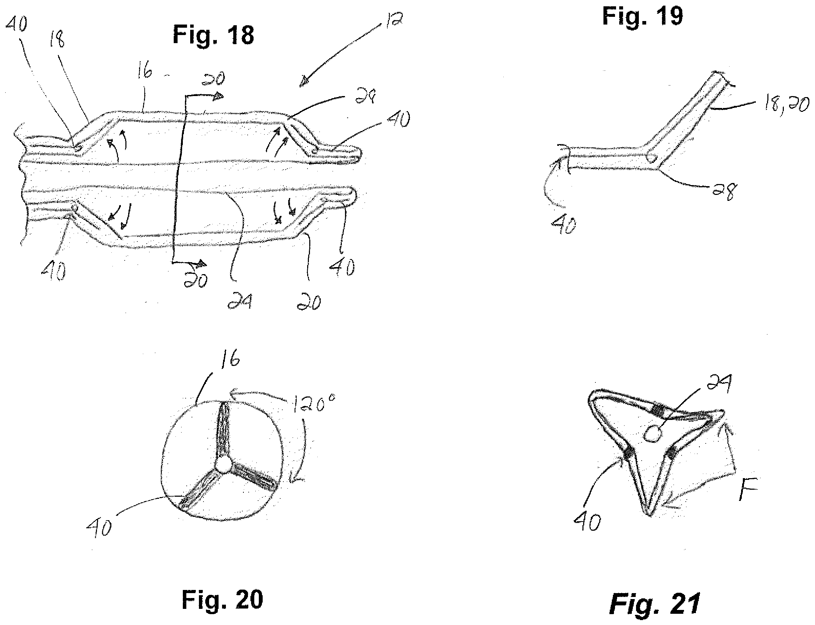

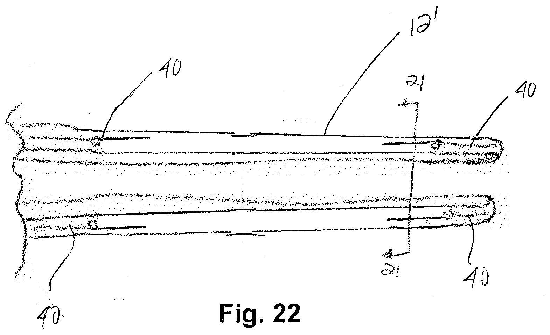

Figures 18-22 illustrate a fourth embodiment of the disclosure.

### Modes for Carrying Out the Invention

The description provided below and in regard to the figures applies to all embodiments unless noted otherwise, and features common to each embodiment are similarly shown and numbered.

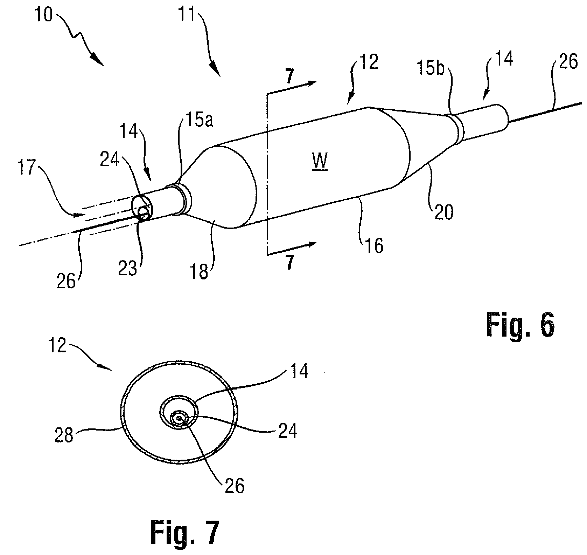

Provided is a catheter 10 having a distal portion 11 with a balloon 12 mounted on a catheter tube 14. Referring to Figures 6, 7, and 8, the balloon 12 has an intermediate section 16, or "barrel," and end sections 18, 20. In one embodiment, the end sections 18, 20 reduce in diameter to join the intermediate section 16 to the catheter tube 14 (and thus sections 18, 20 are generally termed cones or cone sections). The balloon 12 is sealed at balloon ends (proximal end 15a and distal end 15b) on the cone sections 18, 20 to allow the inflation of the balloon 12 via one or more inflation lumens 17 extending within catheter tube 14 and communicating with the interior of the balloon 12.

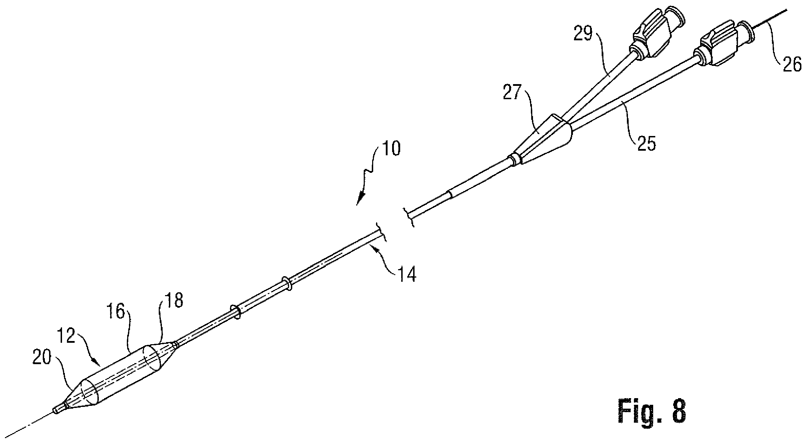

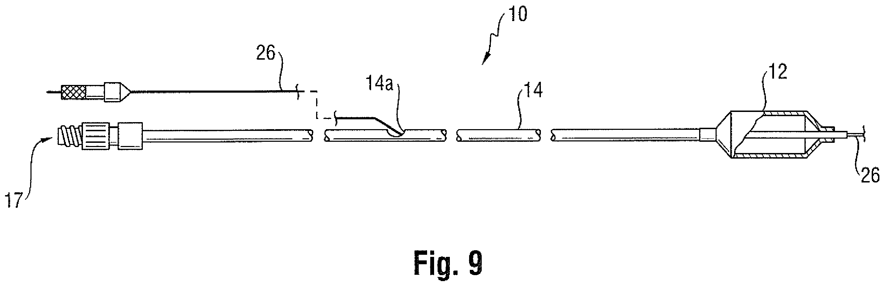

The catheter tube 14 also includes an elongated, tubular shaft 24 forming a guidewire lumen 23 that directs the guidewire 26 through the catheter 10, and along the distal end of which the balloon 12 may be located. As illustrated in Figure 8, this guidewire 26 may extend through the proximal end of the catheter 10 and a first port 25 of a connector 27 into the lumen 23 to achieve an "over the wire" (OTW) arrangement, but could also be provided in a "rapid exchange" (RX) configuration, in which the guidewire 26 exits a lateral opening 14a closer to the distal end (see Figure 9) or else is fed through the tip distally of the balloon 12 (not shown). A second port 29 may also be associated with catheter 10, such as by way of connector 27, for introducing a fluid (e.g., saline, a contrast agent, or both) into the interior compartment of the balloon 12 via the inflation lumen 17.

Balloon 12 may include a single or multi-layered balloon wall 28 forming the interior for receiving the inflation fluid. The balloon 12 may be a non-compliant balloon having a balloon wall 28 that maintains its size and shape in one or more directions when the balloon is inflated. Examples of non-compliant balloons may be found in U.S. Pat. No. 6,746,425 and Publication Nos. US 2006/0085022, US 2006/0085023 and US 2006/0085024. The balloon 12 in such case also has a pre-determined surface area that remains constant during and after inflation, also has a pre-determined length and pre-determined diameter that each, or together, remain constant during and after inflation. However, the balloon 12 could be semi-compliant or compliant instead, depending on the particular use.

In order to provide for enhanced locatability during an interventional procedure, the catheter 10 may be adapted to ensure the alignment between the working surface W and any radiopaque identifier, such as for example the marker bands M on the portion of the shaft 24 passing within the interior of the balloon 12. In one embodiment, this is achieved using a mechanical arrangement for defining the position of one or both ends of the working surface W on inflation of the balloon. For instance, Figures 10 and 11 illustrate an embodiment of a catheter 10 including a balloon 12 carried on a shaft 14 in an inflated and deflated condition. An actuator 30 may be connected at one end to the balloon 12 and at another end to a separate structure, such as the tube 14 or shaft 24. For instance, the actuator 30 may be secured at the distal end 15b of the balloon wall 28, such as between an inner layer 28a and a tying layer 28b of the portion of the wall that forms the conical section 20 of the inflated balloon 12.

In one embodiment, the actuator 30 includes one or more biasing elements, such as springs. The springs may take the form of elongated, thin springs, such as for example an elongated leaf spring 32 that is bent along its longitudinal axis to "snap" between a first, home position and a second, actuated position. As a result, the actuator 30 is adapted for moving in a binary fashion between the first position corresponding to the deflated condition of the balloon 12 (Figure 10) and the second position corresponding to the inflated condition (Figure 11).

When the balloon is deflated, as shown in Figure 10, the actuator 30 may be in a first, non-actuated condition, and lying substantially flat in order to provide the desired low profile for advancing the balloon 12 through the vessel. On inflation, and as shown in Figures 11 and 12, the actuator 30 moves to assume a second, pre-determined position (for spring 32, denoted as 32'), coincident with the erection of the balloon wall 28 (and, in particular, the cone section 20). The fixed second position of the actuator(s) 30 may result in the distal edge or boundary of the working surface W being precisely positioned in alignment with a radiopaque identifier, such as a marker M on the shaft 24.

A similar arrangement may also be provided at the proximal end 15a of the balloon 12, as shown in Figures 13 and 14. In this way, the position of the working surface W may be accurately identified during an interventional procedure by the known alignment with the any radiopaque identifiers intended to correspond to the working surface W, such as markers M on the shaft 24, even upon inflation of the balloon 12.

Moreover, the presence of the actuator(s) 30 also helps to prevent the flattening or pancaking of the balloon 12 on deflation. This is because the folding caused by the evacuation of the contrast agent would cause the actuators 30 to move to the first position, thus helping to ensure the folding of the balloon 12 in the desired manner.

The actuator 30 may be connected in a manner that allows for movement relative to the tip P in moving between the positions. This floating arrangement in one embodiment may be achieved by providing a recess 34 in an extension 35 of the tip P at the distal end 15b of the balloon 12 for receiving and capturing the corresponding free ends 32a of the springs 32, and a corresponding recess 37 adjacent the proximal end 15a of the balloon 12. A single continuous recess may be provided, or individual recesses may be formed as pockets for receiving the individual ends 32a of the springs 32. However, it is possible to reverse the arrangement, such that the end 32a of the spring 32 at the distal end is fixed, and the end 32b corresponding to the balloon 12 is adapted for relative movement in the longitudinal direction.

In an alternative embodiment, the actuators 30, such as springs 32, may be arranged in a similar manner, but with the permitted relative movement being provided by a mated or captured arrangement. Thus, as shown in Figures 15 and 16, the springs 32 may include a distal end 32a having a T-shaped cross-section, and thus having a depending portion 32c (which may form a tongue-in-groove type of arrangement, with a slight taper and a matching arrangement on the groove). This depending portion 32c is adapted for slidably engaging an elongated groove or channel 36 formed along the end of the shaft 24, distally of the cone section 20 of the balloon 12. Specifically, a wall of the shaft 24 may have the channel 36 formed along an outer surface in the longitudinal direction.

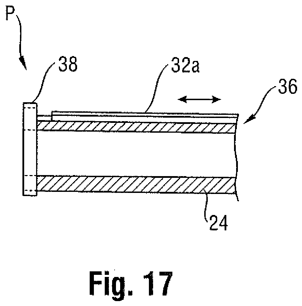

With reference to Figure 17, it may be understood that the arrangement may create a sliding joint. The joint is arranged sufficient clearance such that the captured portion of the spring 32 may freely move within the groove or channel 36, subject to a stop, which may be provided by a cap 38 on the distal tip P of the catheter 10. The proximal end 32b of each spring 32 may be fixed to the cone section 20 of the balloon 12, as shown in Figures 10 and 11.

Instead of or in addition to using traditional markers in the embodiments of Figures 10-13, it may also be possible to instead make the actuator 30, such as spring 32, in a manner such that it is fully or partially radiopaque, so that it thus becomes the radiopaque identifier. This could be done for the embodiment where the proximal ends of the springs 32 are fixed to the balloon 12, such as in Figures 10 and 11. Consequently, the spring 32 on actuation is coincident with the extent of the working surface W and identifiable in view of its radiopaque quality.

Although mention is made of the use of one or more markers M on the shaft 24 to create the radiopaque identifier, it should be appreciated that the disclosure is not limited to any particular form of identifier. Indeed, the radiopaque identifier may be provided independent of the shaft 24, as disclosed in a patent application entitled, **"MEDICAL BALLOON INCLUDING RADIOPAQUE INSERT FOR PRECISELY IDENTIFYING A WORKING SURFACE LOCATION,"** listing as inventors Pat Byrne, Sean Wall, Angela Jensen, Andrew Schaffer, and Angela Crall, U.S. Patent App. Ser. No. 61/608,902, the disclosure of which is incorporated herein by reference.

Balloons 12 that carry one or more surface elements, such as a payload (drug, stent, or both) or a working implement (cutter, focused force wire, or the like) into the vasculature may also benefit from the foregoing description of marking techniques. For example, as shown in Figure 16, a balloon 12 including a defined working surface W may include a portion coated with such a drug D, such as one designed for achieving a desired therapeutic effect when applied to the interior of the vessel. The drug D may be applied to the inflated balloon as part of the manufacturing process, and prior to folding for insertion in the vasculature. The clinician may thus with the benefit of a fluoroscope determine the precise positioning of the working surface W prior to inflating the balloon 12 in the vasculature to deliver the drug D to the desired location and provide the desired treatment regimen.

An actuator may also be used to cause the balloon 12 to collapse to a folded or partially folded state in a pre-determined or preferential manner. Thus, with reference to Figures 18-22, the balloon wall 28 may include one or more actuators in the form of springs. The springs may comprise torsion springs 40 secured to the inside or outside of the balloon wall 28. In one particular embodiment, these torsion springs 40 are provided in the portion of the wall 28 forming one or both of the cone sections 18, 20, and may be embedded therein. In a particular embodiment, the spring or springs 40 may be provided in only the proximal cone section 18, since this is the part that encounters the sheath upon being retracted.

In any case, when the balloon 12 is inflated, as shown in Figure 18, one leg of the spring 40 may extend along the portion of the wall 28 forming the conical section 18 or 20 of the balloon 12. The other leg may extend or project along an adjacent portion of the balloon 12 extending generally parallel to a longitudinal axis of the tube 24. When the balloon 12 is deflated or folded (Figures 21-22), the torsion springs 40 lie substantially flat, and thus do not significantly increase the profile of the balloon 12 in the folded state.

In the illustrated embodiment, as perhaps best understood with reference to Figures 20 and 21, three springs 40 are provided in the wall 28. The springs 40 may be spaced equidistantly apart at 120 degree intervals. However, the relative spacing and hence relative angles could be different, as could the number of springs provided, any of which variables may be adjusted depending on the particular application.

The springs 40 may be biased to provide a force in a direction toward the center of the balloon 12, such as toward tube 24, and thus may urge the balloon 12 to a folded or uninflated condition. When the balloon 12 is inflated, as shown in Figure 18, the pressure is sufficient to overcome the spring force of the spring 40, and thus allow for the desired expansion to the unfolded condition (such as for purposes of dilatation). However, when the pressure against the wall is withdrawn, the springs 40 urge the wall 28 to collapse, returning the balloon 12 to a folded state (12') in a particular manner (e.g., with a tendency for wings or flutes F to form (three in the embodiment with three springs) adjacent to the folds thus formed). This helps to ensure that the balloon 12 folds in a most reliable manner, and thus helps with the repositioning of the deflated balloon (including during any refolding or withdrawal step). However, since the move to the folded state is temporary, re-introducing inflation fluid and the corresponding pressure created would thus overcome the biasing force and inflate the balloon 12 again.

The torsion spring or springs 40 may be made of stainless steel or polymer materials, such as urethane. Shape memory materials that change orientation due to changes in ambient conditions may also be used to help the spring or springs 40 facilitate the folding and unfolding process. The spring or springs 40 may also be made of a bioresorbable or biodegradable material, which may be desirable when placed on an external surface of the balloon wall 28.

While the disclosure presents certain embodiments to illustrate the inventive concepts, numerous modifications, alterations, and changes to the described embodiments are possible without departing from the sphere and scope of the present invention, as defined in the appended claims. For example, the ranges and numerical values provided in the various embodiments are subject to variation due to tolerances, due to variations in environmental factors and material quality, and due to modifications of the structure and shape of the balloon, and thus can be considered to be approximate and the term "approximately" means that the relevant value can, at minimum, vary because of such factors. Accordingly, it is intended that the present disclosure not be limited to the described embodiments, but that it has the full scope defined by the language of the following claims, and equivalents thereof.

## Claims

1. A balloon catheter (10), comprising:
a shaft (24) extending in a longitudinal direction, said shaft having a proximal end and a distal end (18, 20), and supporting at least one radiopaque identifier (M);
an inflatable balloon (12) supported along the distal end of the shaft, the balloon when inflated including a working surface (W); and
an actuator (30) for aligning at least one end of the working surface (W) with the at least one radiopaque identifier (M), wherein
a first portion (32b) of the actuator is
fixed to the balloon (12) and a second portion (32c) of the actuator is adapted for movement relative to the shaft,
wherein the shaft includes a channel (36) for at least partially receiving the second portion of the actuator.

2. The catheter of claim 1, wherein the actuator includes a first position corresponding to a deflated state of the balloon and a second position corresponding to the inflated state of the balloon.

3. The catheter of claim 1 or 2, wherein the actuator comprises a spring (32; 40).

4. The catheter of any of the foregoing claims 1 to 3, wherein the spring comprises a leaf spring (32).

5. The catheter of any of the foregoing claims 1 to 4, wherein the actuator comprises a plurality of springs spaced circumferentially about the catheter.

6. The catheter of claim 1, wherein the first portion of the actuator is captured between two layers on the wall of the balloon.

7. The catheter of any of the foregoing claims, further including a stop (38) for stopping the movement of the actuator.

8. The catheter of any of the foregoing claims, wherein the radiopaque identifier comprises a marker (30) attached to the shaft.

9. The catheter of any of the foregoing claims, wherein the radiopaque identifier comprises an insert positioned within the interior compartment of the balloon.

10. The catheter of any of the foregoing claims, wherein the actuator is a first actuator for aligning a distal end of the working surface with the radiopaque identifier, and further including a second actuator for aligning a proximal end of the working surface with the radiopaque identifier.

11. The catheter of claim 10, wherein each of the first and second actuators comprise a plurality of springs.

12. The catheter of any of the foregoing claims, wherein the radiopaque identifier comprises a first marking and a second marking, and wherein the actuator is a first actuator for aligning a distal end of the working surface with the first marking, and further including a second actuator for aligning a proximal end of the working surface with the second marking.

13. The balloon catheter of any of the foregoing claims, wherein the actuator comprises:
a shaft extending in a longitudinal direction, said shaft having a proximal end and a distal end, and supporting first and second radiopaque identifiers;
a first actuator for aligning a first end of the working surface with the first radiopaque identifier; and
a second actuator for aligning a second end of the working surface with the second radiopaque identifier.

14. The balloon catheter of any of the foregoing claims, comprising:
a shaft for carrying the balloon, the shaft including at least one channel formed in an outer portion of a wall of the shaft; and an actuator having a first end connected to the balloon and a second end at least partially positioned in the channel.

15. The balloon catheter of any of the foregoing claims, comprising:
a shaft for carrying the balloon, the shaft including a plurality of channels (36) formed in an outer portion of the wall of the shaft.

16. The catheter of claim 15, further including an actuator having a first end connected to the balloon and a second end positioned in at least one of the channels.

17. The catheter of any of the foregoing claims, comprising:
a spring connected to a wall of the balloon.

18. The catheter of claim 17, wherein the spring is at least partially radiopaque.

19. The catheter of claim 17 or 18, wherein the spring is connected to a conical section of the wall of the balloon.

## Patentansprüche

1. Ballonkatheter (10), umfassend:
einen Schaft (24), der sich in eine Längsrichtung erstreckt, wobei der Schaft ein proximales Ende und ein distales Ende (18, 20) aufweist, und mindestens eine strahlungsdichte Kennung (M) trägt;
einen aufblasbaren Ballon (12), getragen entlang des distalen Endes des Schafts, wobei der Ballon, wenn aufgeblasen, eine Arbeitsfläche (W) beinhaltet; und
einen Aktuator (30) zu dem Ausrichten mindestens eines Endes der Arbeitsfläche (W) mit der mindestens einen strahlungsdichten Kennung (M), wobei
ein erster Abschnitt (32b) des Aktuators an dem Ballon (12) befestigt ist und ein zweiter Abschnitt (32c) des Aktuators zu der Bewegung bezüglich des Schafts angepasst ist,
wobei der Schaft einen Kanal (36) zu dem mindestens teilweisen Aufnehmen des zweiten Abschnitts des Aktuators enthält.

2. Katheter nach Anspruch 1, wobei der Aktuator eine erste Position, entsprechend einem abgelassenen Zustand des Ballons und eine zweite Position, entsprechend dem aufgeblasenen Zustand des Ballons enthält.

3. Katheter nach Anspruch 1 oder 2, wobei der Aktuator eine Feder (32; 40) umfasst.

4. Katheter nach einem der vorstehenden Ansprüche 1 bis 3, wobei die Feder eine Blattfeder (32) umfasst.

5. Katheter nach einem der vorstehenden Ansprüche 1 bis 4,
wobei der Aktuator eine Vielzahl von Federn umfasst, die umfänglich um den Katheter beabstandet sind.

6. Katheter nach Anspruch 1, wobei der erste Abschnitt des Aktuators zwischen zwei Schichten an der Wand des Ballons eingefangen ist.

7. Katheter nach einem der vorstehenden Ansprüche, der weiter einen Anschlag (38) zu dem Anhalten der Bewegung des Aktuators enthält.

8. Katheter nach einem der vorstehenden Ansprüche, wobei die strahlungsdichte Kennung einen an dem Schaft befestigten Marker (30) umfasst.

9. Katheter nach einem der vorstehenden Ansprüche, wobei die strahlungsdichte Kennung einen in der inneren Kammer des Ballons positionierten Einsatz umfasst.

10. Katheter nach einem der vorstehenden Ansprüche, wobei der Aktuator ein erster Aktuator zu dem Ausrichten eines distalen Endes der Arbeitsfläche mit der strahlungsdichten Kennung ist, und weiter einen zweiten Aktuator zu dem Ausrichten eines proximalen Endes der Arbeitsfläche mit der strahlungsdichten Kennung enthält.

11. Katheter nach Anspruch 10, wobei jeder von den ersten und zweiten Aktuatoren eine Vielzahl von Federn umfasst.

12. Katheter nach einem der vorstehenden Ansprüche, wobei die strahlungsdichte Kennung eine erste Markierung und eine zweite Markierung umfasst, und wobei der Aktuator ein erster Aktuator zu dem Ausrichten eines distalen Endes der Arbeitsfläche mit der ersten Markierung ist, und weiter einen zweiten Aktuator zu dem Ausrichten eines proximalen Endes der Arbeitsfläche mit der zweiten Markierung enthält.

13. Ballonkatheter nach einem der vorstehenden Ansprüche,
wobei der Aktuator umfasst:
einen Schaft, der sich in eine Längsrichtung erstreckt, wobei der Schaft ein proximales Ende und ein distales Ende aufweist, und erste und zweite strahlungsdichte Kennungen trägt;
einen ersten Aktuator zu dem Ausrichten eines ersten Endes der Arbeitsfläche mit der ersten strahlungsdichten Kennung; und
einen zweiten Aktuator zu dem Ausrichten eines zweiten Endes der Arbeitsfläche mit der zweiten strahlungsdichten Kennung.

14. Ballonkatheter nach einem der vorstehenden Ansprüche, umfassend:
einen Schaft zu dem Tragen des Ballons, wobei der Schaft mindestens einen Kanal, der in einem äußeren Abschnitt einer Wand des Schafts ausgebildet ist, enthält; und einen Aktuator, der ein erstes mit dem Ballon verbundenes Ende und ein zweites mindestens teilweise in dem Kanal positioniertes Ende aufweist.

15. Ballonkatheter nach einem der vorstehenden Ansprüche, umfassend:
einen Schaft zu dem Tragen des Ballons, wobei der Schaft eine Vielzahl von Kanälen (36) enthält, die in einem äußeren Abschnitt der Wand des Schafts ausgebildet sind.

16. Katheter nach Anspruch 15, der weiter einen Aktuator enthält, der ein erstes mit dem Ballon verbundenes Ende und ein zweites in mindestens einem der Kanäle positioniertes Ende aufweist.

17. Katheter nach einem der vorstehenden Ansprüche, umfassend:
eine mit einer Wand des Ballons verbundene Feder.

18. Katheter nach Anspruch 17, wobei die Feder mindestens teilweise strahlungsdicht ist.

19. Katheter nach Anspruch 17 oder 18, wobei die Feder mit einem konischen Abschnitt der Wand des Ballons verbunden ist.

## Revendications

1. Cathéter à ballonnet (10), comprenant :
une tige (24) s'étendant dans une direction longitudinale, ladite tige ayant une extrémité proximale et une extrémité distale (18, 20), et supportant au moins un identificateur radio-opaque (M),
un ballonnet gonflable (12) supporté le long de l'extrémité distale de la tige, le ballonnet, lorsqu'il est gonflé, incluant une surface de travail (W) ; et
un actionneur (30) pour aligner au moins une extrémité de la surface de travail (W) avec le au moins un identificateur radio-opaque (M), dans lequel
une première partie (32b) de l'actionneur est fixée au ballonnet (12) et une seconde partie (32c) de l'actionneur est adaptée pour se déplacer par rapport à la tige,
dans lequel la tige inclut un canal (36) pour recevoir au moins partiellement la seconde partie de l'actionneur.

2. Cathéter selon la revendication 1, dans lequel l'actionneur inclut une première position correspondant à un état dégonflé du ballonnet et une seconde position correspondant à un état gonflé du ballonnet.

3. Cathéter selon la revendication 1 ou 2, dans lequel l'actionneur comprend un ressort (32 ; 40).

4. Cathéter selon l'une quelconque des revendications précédentes 1 à 3, dans lequel le ressort comprend un ressort à lames (32).

5. Cathéter selon l'une quelconque des revendications précédentes 1 à 4, dans lequel l'actionneur comprend une pluralité de ressorts espacés circonférentiellement autour du cathéter.

6. Cathéter selon la revendication 1, dans lequel la première partie de l'actionneur est capturée entre deux couches sur la paroi du ballonnet.

7. Cathéter selon l'une quelconque des revendications précédentes, comprenant en outre une butée (38) pour arrêter le mouvement de l'actionneur.

8. Cathéter selon l'une quelconque des revendications précédentes, dans lequel l'identificateur radio-opaque comprend un marqueur (30) attaché à la tige.

9. Cathéter selon l'une quelconque des revendications précédentes, dans lequel l'identificateur radio-opaque comprend un insert positionné dans le compartiment intérieur du ballonnet.

10. Cathéter selon l'une quelconque des revendications précédentes, dans lequel l'actionneur est un premier actionneur pour aligner une extrémité distale de la surface de travail avec l'identificateur radio-opaque, et incluant en outre un second actionneur pour aligner une extrémité proximale de la surface de travail avec l'identificateur radio-opaque.

11. Cathéter selon la revendication 10, dans lequel chacun des premier et second actionneurs comprend une pluralité de ressorts.

12. Cathéter selon l'une quelconque des revendications précédentes, dans lequel l'identificateur radio-opaque comprend un premier marquage et un second marquage, et dans lequel l'actionneur est un premier actionneur pour aligner une extrémité distale de la surface de travail avec le premier marquage, et incluant en outre un second actionneur pour aligner une extrémité proximale de la surface de travail avec le second marquage.

13. Cathéter à ballonnet selon l'une quelconque des revendications précédentes, dans lequel l'actionneur comprend :
une tige s'étendant dans une direction longitudinale, ladite tige ayant une extrémité proximale et une extrémité distale, et supportant des premier et second identificateurs radio-opaques ;
un premier actionneur pour aligner une première extrémité de la surface de travail avec le premier identificateur radio-opaque ; et
un second actionneur pour aligner une seconde extrémité de la surface de travail avec le second identificateur radio-opaque.

14. Cathéter à ballonnet selon l'une quelconque des revendications précédentes, comprenant :
une tige pour porter le ballonnet, la tige incluant au moins un canal formé dans une partie extérieure d'une paroi de la tige ; et un actionneur ayant une première extrémité connectée au ballonnet et une seconde extrémité au moins partiellement positionnée dans le canal.

15. Cathéter à ballonnet selon l'une quelconque des revendications précédentes, comprenant :
une tige pour porter le ballonnet, la tige incluant une pluralité de canaux (36) formés dans une partie externe de la paroi de la tige.

16. Cathéter selon la revendication 15, incluant en outre un actionneur ayant une première extrémité connectée au ballonnet et une seconde extrémité positionnée dans au moins un des canaux.

17. Cathéter à ballonnet selon l'une quelconque des revendications précédentes, comprenant :
un ressort connecté à une paroi du ballonnet.

18. Cathéter selon la revendication 17, dans lequel le ressort est au moins partiellement radio-opaque.

19. Cathéter selon la revendication 17 ou 18, dans lequel le ressort est connecté à une section conique de la paroi du ballonnet.
